# EUROPÄISCHE PATENTSCHRIFT

(11) **EP 0 983 746 B1**
(45) Veröffentlichungstag und Bekanntmachung des Hinweises auf die Patenterteilung: **19.03.2003**
(21) Anmeldenummer: 99116605.9
(22) Anmeldetag: 25.08.1999
(51) Int. Cl.: A61B 5/20, A61F 5/44

(54) **Urinmessgerät**
Device for measuring urine
Appareil de mesure d'urine

(30) Priorität: 02.09.1998 DE 19839962
(43) Veröffentlichungstag der Anmeldung: 08.03.2000
(73) Patentinhaber: B. BRAUN MELSUNGEN AG, 34212 Melsungen (DE)
(72) Erfinder: Sippel, Martin, Dr., 34212 Melsungen (DE); Collin, Rémi, 28230 Epernon (FR); Voges, Karl-Friedrich, 34212 Melsungen (DE)
(74) Vertreter: Selting, Günther, Dipl.-Ing.

(56) Entgegenhaltungen:
- EP-A- 0 247 958
- DE-A- 3 837 857
- GB-A- 2 322 079

## Beschreibung

Die Erfindung betrifft ein Urinmeßgerät, das den von einem Patienten kommenden Urinfluß aufnimmt und bereithält, um beispielsweise die Urinmenge zu kontrollieren oder Urinproben verfügbar zu machen.

Urinmeßgeräte weisen üblicherweise einen am Bett des Patienten zu befestigenden Halter auf. An diesem Halter ist ein starrer Meßbehälter befestigt, in den ein vom Patienten kommender Schlauch einmündet. Der Meßbehälter enthält eine oder mehrere Meßkammern, die zumeist durchsichtig sind und eine Meßskala aufweisen, wodurch die in der Meßkammer befindliche Urinmenge abgelesen werden kann. Von der Meßkammer führt ein Überlaufanschluß zu einem Auffangbeutel. Dieser Auffangbeutel ist auswechselbar an der Meßkammer oder am Halter befestigt und er kann bei Bedarf vom Halter abgenommen und entleert werden. Bevor der Auffangbeutel entleert wird, sollte zunächst der Inhalt des Meßbehälters in den Auffangbeutel überführt werden. Hierzu kann der Meßbehälter gekippt bzw. schräggestellt werden, so daß sein Inhalt in den Auffangbeutel abläuft. Sofern die bekannten Urinmeßgeräte ein Kippen des Meßbehälters ermöglichen, muß der Meßbehälter zum Kippen zunächst aus dem Halter ausgehängt und anschließend wieder eingehängt werden.

Bei einem aus DE 38 37 857 A1 bekannten Urinmeßgerät ist der normalerweise senkrecht hängende Meßbehälter zum Entleeren seines Inhalts in den Auffangbeutel schwenkbar angebracht. Beim Hochschwenken des Meßbehälters entleert sich dieser in den Auffangbeutel. Hierbei ist eine flexible Verbindung zwischen Meßbehälter und Auffangbeutel erforderlich. Der Auffangbeutel ist bleibend mit dem Messbehälter verbunden, so dass er nicht separat auswechselbar ist.

In US 5,409,014 ist ein Urinmessgerät beschrieben, bei dem der Messbehälter und der Auffangbeutel jeweils eine Aufhängeöse aufweisen, um sie an einem Haken aufzuhängen. Ein spezieller Halter, der den Auffangbeutel festhält, während dieser mit dem Überlauf des Messbehälters verbunden ist, ist nicht vorgesehen. Der vom Patienten kommende Zulaufschlauch wird mit dem Einlass des Messbehälters über eine Steckverbindung aus männlichem und weiblichem Verbindungsteil verbunden. In ähnlicher Weise wird der Einlass des Auffangbeutels mit dem Auslaufrohr des Messbehälters verbunden. Der Messbehälter kann zum Entleeren gekippt werden.

Ein Urinmessgerät, von dem der Oberbegriff des Patentanspruchs 1 ausgeht, ist bekannt aus GB 2 322 079 A. Bei diesem Urinmessgerät ist der Messbehälter mit dem Halter durch eine Rohrkupplung verbunden, die ein Verschwenken des Messbehälters um eine rechtwinkelig zu der Ebene des Halters verlaufende Achse ermöglicht. Durch derartiges Verschwenken kann der Inhalt des Messbehälters in einen an dem Halter befestigten Beutel entleert werden. Eine vollständige Restentleerung des Messbehälters ist sehr schwer zu erreichen.

EP 0 247 958 A1 beschreibt ein Urinmessgerät mit einem Halter, in dem ein fester Behälter integriert ist. An dem Halter ist ein schwenkbarer Behälter befestigt, der um eine parallel zur Ebene des Halters verlaufende Achse herum hochschwenkbar ist. Beide Behälter sind durch eine Rohrkupplung miteinander verbunden, durch welche beim Hochschwenken des Messbehälters der Urin aus diesem abfließt. Die Schwenkachse verläuft schräg zur Horizontalen.

Der Erfindung liegt die Aufgabe zugrunde, ein Urinmessgerät zu schaffen, das ein einfaches Entleeren des Messbehälters in den Auffangbeutel ermöglicht und für das Krankenhauspersonal bedienungsfreundlich ist.

Die Lösung dieser Aufgabe erfolgt erfindungsgemäß mit den im Patentanspruch 1 angegebenen Merkmalen.

Bei dem erfindungsgemäßen Urinmessgerät ist der Überlaufanschluss des Messbehälters mit einem Verbindungsrohr des Halters durch eine Rohrkupplung verbunden. Diese Rohrkupplung verläuft in der Schwenkachse, um die der Messbehälter relativ zu dem Halter schwenkbar ist. Die Rohrkupplung bildet ein Drehgelenk, das den Messbehälter unabhängig von seiner Schwenkstellung jeweils direkt mit dem Halter verbindet. Somit ist in jeder Schwenkstellung der Meßbehälter in bezug auf den Halter definiert geführt, unter Beibehaltung der Fließverbindung zwischen Meßbehälter und Verbindungsrohr bzw. Auffangbeutel. Zum Entleeren des Meßbehälters braucht der Meßbehälter lediglich um seine Schwenkachse herum hochgeschwenkt zu werden. Daraufhin läuft der Urin von dem Meßbehälter durch die Rohrkupplung in den Auffangbeutel. Vorzugsweise ist die Reibungskraft im Schwenkgelenk so eingestellt, daß der Meßbehälter in jeder Stellung stehenbleibt. Er sollte jedoch mindestens in der Auslaufstellung, in der sein Inhalt vollständig ausfließen kann, rastend oder reibend festgehalten werden. Nach der Entleerung wird der Meßbehälter wieder in die Ausgangslage zurückgeschwenkt. Dabei braucht er nicht in den Halter eingehängt zu werden; es genügt ein einfaches Herunterklappen.

Ein vollständiges Leerlaufen des Meßbehälters im hochgeschwenkten Zustand wird erleichtert, wenn die Schwenkachse schräg zu einer Horizontalen des Halters verläuft, so daß der Auslauf sich im Bereich der tiefsten Stelle des hochgeschwenkten Meßbehälters befindet.

Gemäß einer bevorzugten Ausgestaltung der Erfindung ist vorgesehen, daß der Meßbehälter in der Gebrauchsstellung, also im heruntergeschwenkten Zustand, den Halter und einen Teil des Auffangbeutels bedeckt oder mindestens teilweise verdeckt. Wenn der Halter vollständig verdeckt ist, sind bei frontaler Betrachtung nur der Meßbehälter und der Auffangbeutel sichtbar, also nur diejenigen Teile, die einer Kontrolle durch das Personal bedürfen. Der Halter, der lediglich eine Hilfsfunktion hat, sowie die Befestigungsmittel zur Anbringung des Halters am Krankenbett oder einer anderen Tragvorrichtung werden verdeckt. Der Halter ist jedoch nach dem Hochschwenken des Meßbehälters einsehbar und zugänglich.

Der Meßbehälter wird zum Entleeren um eine feste Achse des Halters herum hochgeschwenkt. Diese Schwenkachse verläuft vorzugsweise parallel zu der Ebene des Halters oder derjenigen des am Halter aufgehängten Auffangbeutels. Es ist jedoch auch möglich, den Meßbehälter um eine Schwenkachse zu schwenken, die senkrecht oder unter einem anderen Winkel zur Ebene des Halters verläuft.

Bei einer bevorzugten Ausführungsform des Urinmeßgerätes ist längs der Schwenkachse eine irreversible Rastkupplung angeordnet.

Die Erfindung betrifft ferner eine verbesserte Ankupplung des Auffangbeutels an den Halter. Hierzu sind der Halter und der Einlaufstutzen des Auffangbeutels mit Verriegelungsmitteln ausgestattet, durch die der Einlaufstutzen unter Drehung um weniger als 120° um seine Achse an einem Verbindungsrohr des Halters verriegelbar ist. Die Verbindung zwischen dem Halter und dem Auffangbeutel dient nicht nur der Überleitung der Flüssigkeit, sondern trägt auch dazu bei, das Beutelgewicht zu tragen. Durch die Verriegelung des Einlaufstutzens am Verbindungsrohr wird eine Art vergrößerte Luer-Lock-Verbindung geschaffen, wodurch ein Ablösen des Auffangbeutels von dem Halter unter dem Einfluß des Beutelgewichts verhindert wird. Durch die Zusammenfassung der Funktionen des Tragens und des Überleitens in einem einzigen Bauteil wird ein zusätzliches Befestigungselement am Halter zum Tragen des Beutels eingespart. Außerdem wird der Beutelwechsel beschleunigt.

Im folgenden werden unter Bezugnahme auf die Zeichnungen Ausführungsbeispiele der Erfindung näher erläutert.

Es zeigen:
- Fig. 1: eine Frontansicht des Urinmeßgeräts,
- Fig. 2: eine Rückansicht des Urinmeßgeräts,
- Fig. 3: eine Seitenansicht aus Richtung des Pfeiles III von Fig. 2, und
- Fig. 4: eine Rückansicht des Urinmeßgeräts, wobei der Meßbehälter zum Entleeren hochgeklappt ist, aus Richtung des Pfeiles IV von Fig. 3.

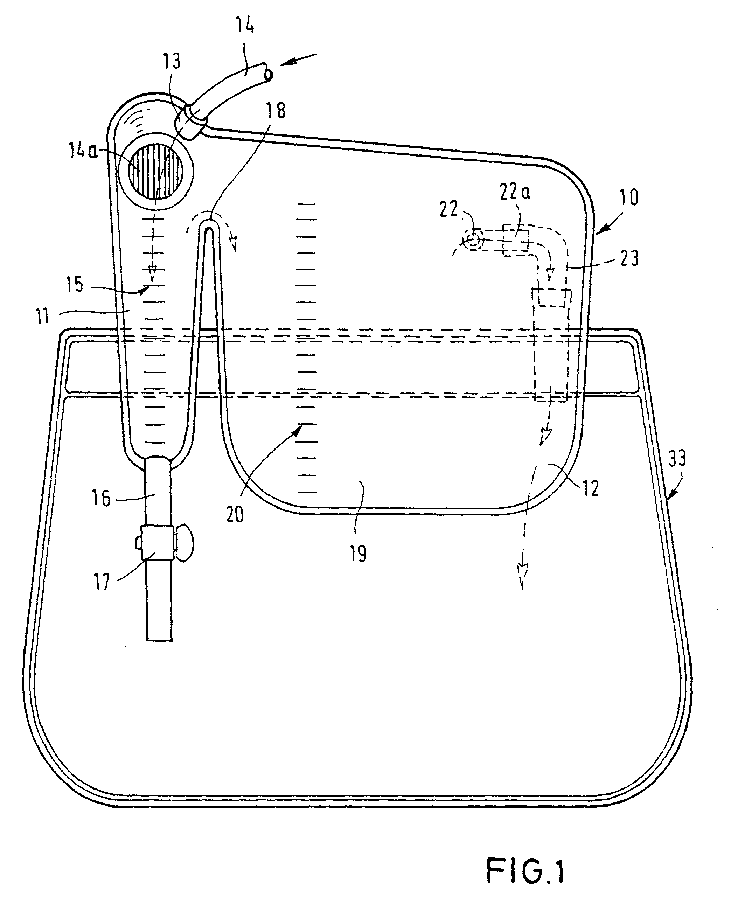

Das in den Fign. 1-4 dargestellte Ausführungsbeispiel eines Urinmeßgeräts weist einen Meßbehälter 10 auf, der aus Kunststoff besteht, wobei mindestens die Vorderseite durchsichtig ist. Der Meßbehälter 10 ist ein Flachbehälter, der eine kleinere erste Meßkammer 11 und eine größere zweite Meßkammer 19 aufweist. In die erste Meßkammer 11 führt ein Zulaufanschluß 13 hinein, der mit einem vom Patienten kommenden Schlauch 14 verbunden ist. Der Zulaufanschluß ist mit einer Entlüftungskammer verbunden, die eine Entlüftungsöffnung 14a aufweist. Von dieser Entlüftungskammer führt ein Überlauf in die erste Meßkammer 11. Die erste Meßkammer 11 ist nach Art eines vertikalen Schachtes ausgebildet und sie weist eine Skala 15 auf, an der der Flüssigkeitsstand abgelesen werden kann. Zur Entnahme von Urinproben ist das untere Ende der Meßkammer 11 mit einem Auslaufschlauch 16 verbunden, an dem ein Ventilhahn 17 angebracht ist.

Von der ersten Meßkammer 11 führt ein Überlauf 18 zu der größeren zweiten Meßkammer 19. Die Vorderwand dieser Meßkammer 19 weist ebenfalls eine Skala 20 auf, an der der Flüssigkeitsstand abgelesen werden kann. Beide Meßkammern 11,19 bilden zusammen den Meßbehälter 10.

Von der Rückwand 21 der zweiten Meßkammer 19 geht ein Überlaufanschluß 22, der der Rückwand 21 integral angeformt ist, nach hinten ab. Dieser Überlaufanschluß 22 besteht aus einem rechtwinklig gebogenen Rohrkrümmer mit einem Rohrstück 22a, welches parallel zu der Rückwand 21 verläuft.

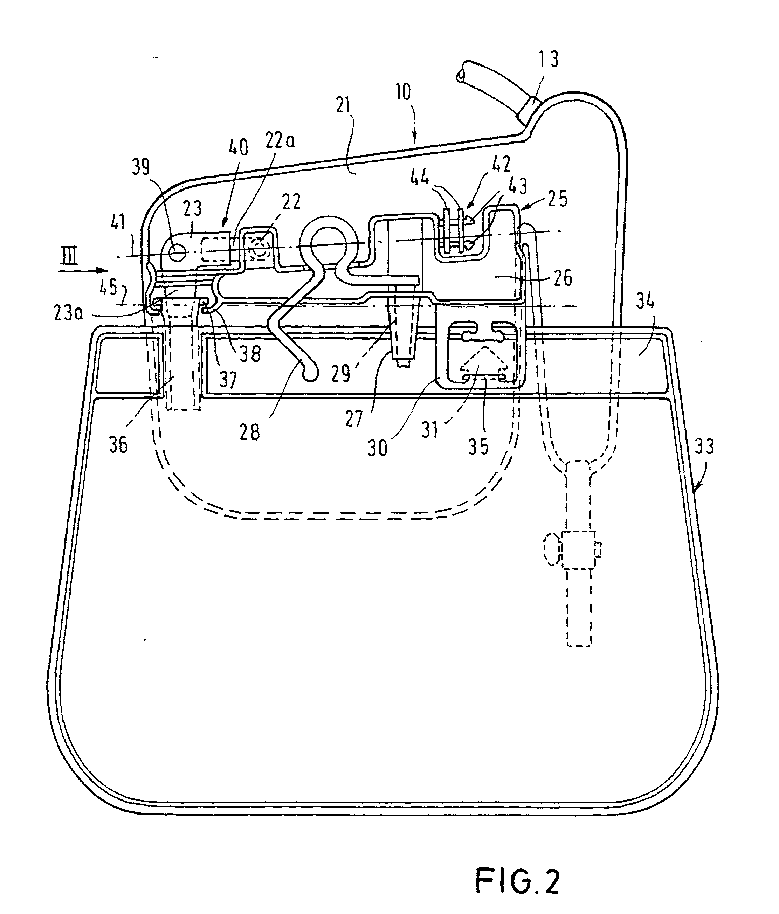

Der Meßbehälter 10 ist an einem Halter 25 angebracht, der seinerseits an einem Krankenbett, einem Gestell oder an einer anderen Tragvorrichtung angebracht werden kann. Der Halter 25 weist gemäß Fig. 2 eine mit einem Verstärkungsrand versehene Tragplatte 26 auf. An dieser Tragplatte 26 ist eine erste Befestigungsvorrichtung 27 in Form eines Steckzapfens und eine zweite Befestigungsvorrichtung 28 in Form eines elastischen Hakens angebracht. Die Befestigungsvorrichtung 28 kann um ihre in den Steckzapfen eingreifende Achse 29 herum geschwenkt werden. An der Unterseite des Halters 25 befindet sich eine Aufhängevorrichtung 30 in Form einer Tragöse, die einen nach oben aufragenden Dorn 31 aufweist.

An dem Verbindungsrohr 23 und der Aufhängevorrichtung 30, die beide Bestandteil des Halters 25 sind, ist der Auffangbeutel 33 aufgehängt. Dieser Beutel ist ein flexibler Folienbeutel, der den Urin aufnimmt, welcher aus dem Überlaufanschluß 22 des Meßbehälters 10 abläuft. Der Auffangbeutel 33 ist an seinem oberen Ende mit einem durch Siegelungslinien begrenzten Verstärkungsrand 34 versehen. Im Verstärkungsrand 34 befindet sich ein Schlitz 35, durch den der Dorn 31 der Aufhängevorrichtung 30 hindurchgesteckt wird. Ferner verläuft durch den Verstärkungsrand 34 ein rohrförmiger Einlaufstutzen 36. Während der Auffangbeutel 33 aus flexibler Folie besteht, besteht der Einlaufstutzen 36 aus einem relativ steifen Kunststoffrohr. Der Einlaufstutzen 36 wird über den nach unten weisenden Schenkel 23a des Verbindungsrohrs 23 geschoben. An der Außenseite der Einlauföffnung des Einlaufstutzens 36 befinden sich Verriegelungsmittel 37 in Form abstehender Vorsprünge und an dem Halter 25 befinden sich ebenfalls Verriegelungsmittel 38. Die Verriegelungsmittel 37,38 wirken nach Art einer Luer-Lock-Verbindung zusammen und sie halten den Einlaufstutzen 36 gegen Abziehen von dem Rohrschenkel 23a fest. Der Rohrschenkel 23a ist als Außenkonus ausgebildet und das obere Ende des Einlaufstutzens 36 ist als Innenkonus ausgebildet. Die Verriegelung erfolgt dadurch, daß der Einlaufstutzen 36 zunächst um etwa 90° verdreht auf den Rohrschenkel 23a teleskopisch aufgeschoben wird, bis der Einlaufstutzen 36 in dichtenden Eingriff mit dem Rohrstutzen 23a gelangt. Dann wird der Einlaufstutzen 36 um 90° zurückgedreht, wobei die Verriegelungsmittel 37,38 in den Verriegelungszustand gelangen. Diese Schlauchverbindung bildet also zugleich eine Tragverbindung, die zusammen mit der Aufhängevorrichtung 30 den Auffangbeutel 33 trägt. In dem Verbindungsrohr 23 ist eine Entlüftungsvorrichtung mit Entlüftungsöffnung 39 zur Entlüftung des Auffangbeutels 33 vorgesehen.

Der Rohrstutzen 22a des Überlaufanschlusses 22 bildet zusammen mit dem Verbindungsrohr 23 des Halters 25 eine als Drehkupplung wirkende Rohrkupplung 40. Die Achse dieser Rohrkupplung 40 bildet die Schwenkachse 41, um die herum der Meßbehälter 10 relativ zu dem Halter 25 geschwenkt werden kann. Auf der Schwenkachse 41 liegt ferner eine Rastkupplung 42. Diese besteht aus federnden Dornen 43 des Halters 25, die durch Ringe 44 des Meßbehälters 10 hindurchgesteckt und verrastet sind. Die Ringe 44 können um die Dorne 43 herum gedreht werden, so daß die Rastkupplung 42 ein entlang der Schwenkachse 41 angeordnetes Drehlager bildet. Die Schwenkachse 41 verläuft parallel zu der (vertikalen) Ebene des Halters 26. Wenn der Halter 26 exakt horizontal ausgerichtet ist, verläuft die Schwenkachse 41 unter einem geringen Winkel zur Horizontalen 45, wobei sie von der Rohrkupplung 40 bzw. dem Überlaufanschluß 22 aus ansteigt. Der Überlaufanschluß 22 befindet sich in dem von dem Zulaufanschluß 13 abgewandten Bereich des Meßbehälters.

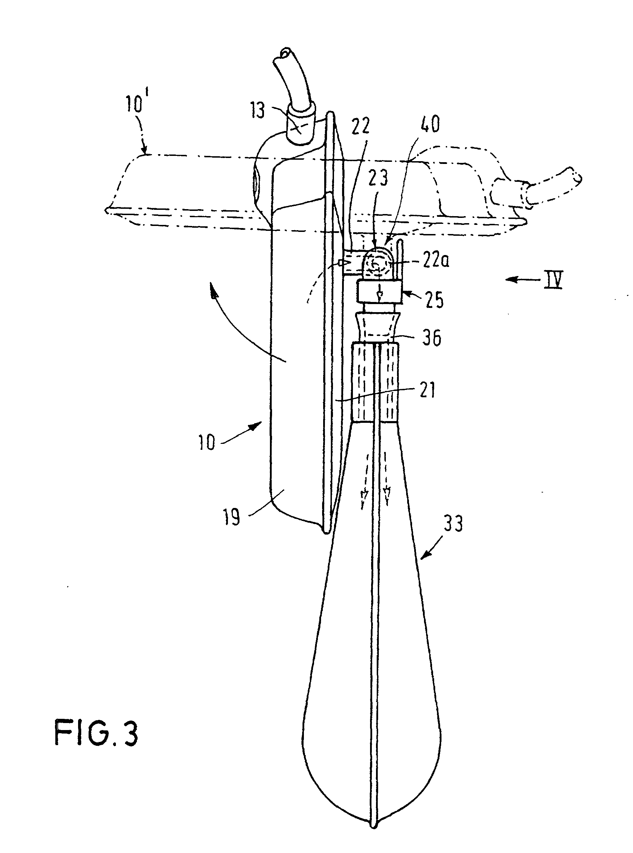

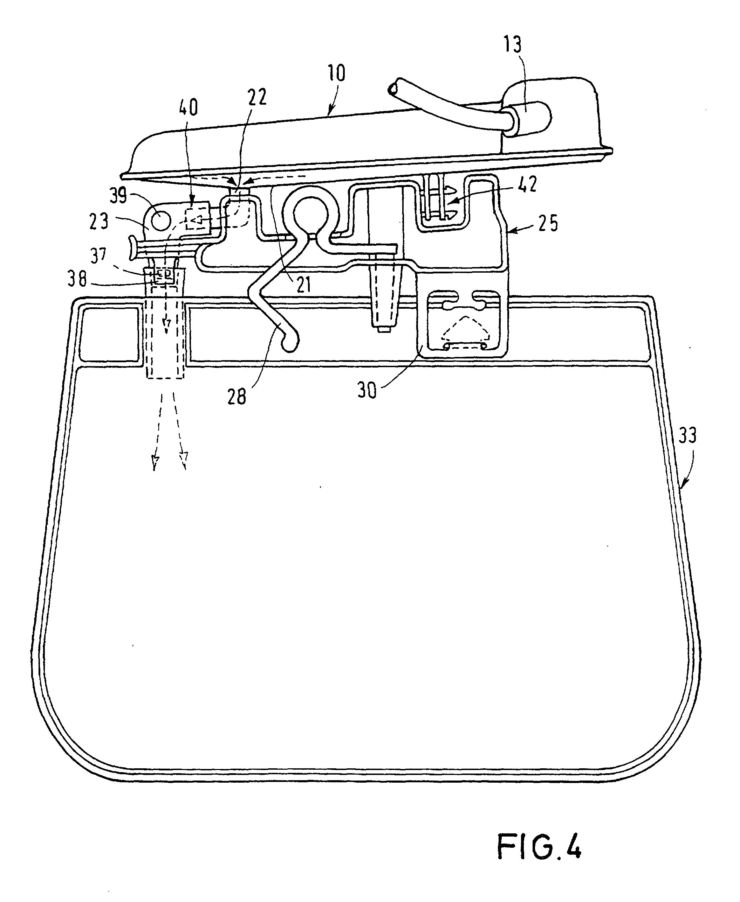

Wenn der Meßbehälter 10 um die Schwenkachse 41 herum hochgeschwenkt wird, wie dies in Fig. 3 gestrichelt und in Fig. 4 in durchgezogenen Linien dargestellt ist, befindet sich der Überlaufanschluß 22 an der tiefsten Stelle. Zu dieser Stelle hin hat die Rückwand 21 ein Gefälle. Daher kann der Inhalt des Meßbehälters 10 zum Überlaufanschluß 22 hin ablaufen, und zwar aus der ersten Meßkammer 11 und der zweiten Meßkammer 19 heraus. Durch die Rohrkupplung 40 fließt der Urin dann in das Verbindungsrohr 23 und von diesem in den Auffangbeutel 33.

Die Fign. 1 und 3 zeigen die Gebrauchsstellung des Urinmeßgerätes, in der der Urin zunächst in die erste Meßkammer 11 einläuft, um von dieser dann über den Überlauf 18 in die zweite Meßkammer 19 zu fließen. Wenn die zweite Meßkammer 19 gefüllt ist, fließt weiterer zulaufender Urin durch den Überlaufanschluß 22 in den Auffangbeutel 33.

Zum Entleeren des Meßbehälters 10 wird dieser um etwa 90° hochgeschwenkt, so daß er die in Fig. 3 mit 10' bezeichnete Stellung einnimmt. In dieser Stellung läuft der Urin aus dem gesamten Meßbehälter 10 durch die Rohrkupplung 40 in den Auffangbeutel 33 ab. Danach kann der Auffangbeutel 33 von dem Halter 26 abgenommen und gegen einen leeren Auffangbeutel ausgetauscht werden. In der hochgeschwenkten Position bleibt der Meßbehälter 10 durch Reibung oder Rastung stehen, d.h. er braucht während des Ablaufvorganges nicht vom Personal festgehalten zu werden. Danach wird er in die Gebrauchsstellung zurückgeschwenkt.

## Patentansprüche

1. Urinmessgerät mit einem Halter (25), einem an dem Halter (25) befestigten Messbehälter (10), der einen Zulaufanschluss (13) und einen Überlaufanschluss (22) aufweist und an dem Halter (25) um eine Schwenkachse (41) schwenkbar ist, einem mit dem Überlaufanschluss (22) des Messbehälters verbundenen, am Halter (25) aufgehängten Auffangbeutel (33) und einer längs der Schwenkachse (41) angeordneten Rohrkupplung (40), die den Überlaufanschluss (22) des Messbehälters (10) drehbar mit einem Verbindungsrohr (23) des Halters (25) verbindet, wobei an das Verbindungsrohr (23) der Auffangbeutel (33) anschließbar ist,
**dadurch gekennzeichnet,**
**dass** die Schwenkachse (41) im Abstand von dem Messbehälter (10) angeordnet ist, dass vom Messbehälter (10) ein den Überlaufanschluss (22) bildender Rohrkrümmer absteht, dessen abgewinkelter Rohrstutzen (22a) mit einem Verbindungsrohr (23) des Halters (25) die Rohrkupplung (40) bildet, und dass der Messbehälter (10) mit dem Halter (25) außerdem durch eine auf der Schwenkachse (41) liegende, ein Drehlager bildende Rastkupplung (42) verbunden ist.

2. Urinmessgerät nach Anspruch 1, **dadurch gekennzeichnet, dass** die Schwenkachse (41) schräg zu einer Horizontalachse (45) des Halters (25) verläuft.

3. Urinmessgerät nach Anspruch 1 oder 2, **dadurch gekennzeichnet, dass** der Messbehälter (10) um mindestens etwa 90° hochschwenkbar ist.

4. Urinmessgerät nach einem der Ansprüche 1-3, **dadurch gekennzeichnet, dass** der Messbehälter (10) in der Gebrauchsstellung bei frontaler Betrachtung den Halter (25) und einen Teil des Auffangbeutels (33) verdeckt.

5. Urinmessgerät nach einem der Ansprüche 1-4, **dadurch gekennzeichnet, dass** die Schwenkachse (41) parallel zu der Ebene einer Tragplatte (26) des Halters (25) verläuft.

6. Urinmessgerät nach einem der Ansprüche 1-5, **dadurch gekennzeichnet, dass** das Verbindungsrohr (23) des Halters (25) ein im Wesentlichen rechtwinkeliges Rohrknie aufweist, in dem eine Entlüftungsöffnung (39) angeordnet ist.

7. Urinmessgerät nach einem der Ansprüche 1-6, **dadurch gekennzeichnet, dass** der Messbehälter (10) im hochgeschwenkten Zustand reibend oder rastend festgehalten wird.

8. Urinmessgerät nach einem der Ansprüche 1-7, **dadurch gekennzeichnet, dass** der Halter (25) und ein Einlaufstutzen (36) des Auffangbeutels (33) mit Verriegelungsmitteln (37,38) ausgestattet sind, durch die der Einlaufstutzen (36) unter Drehung um weniger als 120° um seine Achse an dem Verbindungsrohr (23) verriegelbar ist.

## Claims

1. Urine measuring device comprising a holder (25), a measuring container (10) attached to said holder (25), the measuring container (10) being provided with a supply connection (13) and an overflow connection (22) and being pivotable about a pivoting axis (41) at the holder (25), a collecting bag (33) connected with the overflow connection (22) of the measuring container and attached to the holder (25), and a pipe coupling (40) arranged along the pivoting axis (41) and rotatably connecting the overflow connection (22) of the measuring container (10) with a connecting pipe (23) of the holder (25), wherein the collecting bag (33) is connectable to the connecting pipe (23),
**characterized in that**
the pivoting axis (41) is arranged in spaced relationship to the measuring container (10), a pipe bend forming the overflow connection (22) extends from the measuring container (10), the angled pipe stud (22a) of the pipe bend forming, together with a connecting pipe (23) of the holder (25), the pipe coupling (40), and the measuring container (10) is further connected with the holder (25) by a snap-in coupling (42) lying on the pivoting axis (41) and forming a pivot bearing.

2. Urine measuring device according to claim 1, **characterized in that** the pivoting axis (41) extends transversally to a horizontal axis (45) of the holder (25).

3. Urine measuring device according to claim 1 or 2, **characterized in that** the measuring container (10) is pivotable upwardly by at least approximately 90°.

4. Urine measuring device according to one of claims 1-3, **characterized in that**, as seen from the front, the measuring container (10), in its position of use, covers the holder (25) and part of the collecting bag (33).

5. Urine measuring device according to one of claims 1-4, **characterized in that** the pivoting axis (41) extends in parallel to the plane of the carrier plate (26) of the holder (25).

6. Urine measuring device according to one of claims 1-5, **characterized in that** the connecting pipe (23) of the holder (25) comprises a substantially right-angled elbow in which a vent hole (39) is arranged.

7. Urine measuring device according to one of claims 1-6, **characterized in that** the measuring container (10) is retained by friction or catching in the swung-up position.

8. Urine measuring device according to one of claims 1-7, **characterized in that** the holder (25) and an inlet branch (36) of the collecting bag (33) are provided with locking means (37,38) by means of which the inlet branch (36) is lockable at the connecting pipe (23) by being turned by less than 120° about its axis.

## Revendications

1. Appareil de mesure pour urine comprenant un support (25) et un récipient de mesure (10) qui est fixé au support (25), qui comporte un raccord d'entrée d'écoulement (13) ainsi qu'un raccord de trop plein (22) et peut pivoter sur le support (25) autour d'un axe de pivotement (41), l'appareil comprenant également une poche de collecte (33) accrochée sur le support (25) et reliée au raccord de trop plein (22) du récipient de mesure, ainsi qu'un accouplement de tube (40) qui est disposé le long de l'axe de pivotement (41) et relie de manière rotative le raccord de trop plein (22) du récipient de mesure (10) à un tube de liaison (23) du support (25), la poche de collecte (33) pouvant être raccordée au tube de liaison (23), **caractérisé en ce que** l'axe de pivotement (41) est disposé à distance du récipient de mesure (10), **en ce que** du récipient de mesure (10) fait saillie un tube coudé formant le raccord de trop plein (22) et dont l'embout tubulaire coudé (22a) forme avec un tube de liaison (23) du support (25), l'accouplement de tube (40), et **en ce que** le récipient de mesure (10) est relié en outre au support (25) par un accouplement par encliquetage (42) formant un palier de rotation et situé sur l'axe de pivotement (41).

2. Appareil de mesure pour urine selon la revendication 1, **caractérisé en ce que** l'axe de pivotement (41) s'étend de manière oblique par rapport à un axe horizontal (45) du support (25).

3. Appareil de mesure pour urine selon la revendication 1 ou 2, **caractérisé en ce qu'**il est possible de faire pivoter le récipient de mesure (10) vers le haut d'au moins environ 90°.

4. Appareil de mesure pour urine selon l'une des revendications 1-3, **caractérisé en ce que** le récipient de mesure (10), dans la position d'utilisation, en l'observant de face, recouvre le support (25) et une partie de la poche de collecte (33).

5. Appareil de mesure pour urine selon l'une des revendications 1-4, **caractérisé en ce que** l'axe de pivotement (41) s'étend parallèlement au plan d'une plaque de support (26) du support (25).

6. Appareil de mesure pour urine selon l'une des revendications 1-5, **caractérisé en ce que** le tube de liaison (23) du support (25) présente un coude tubulaire sensiblement à angle droit, dans lequel est disposée une ouverture de purge d'air (39).

7. Appareil de mesure pour urine selon l'une des revendications 1-6, **caractérisé en ce que** le récipient de mesure (10) est maintenu dans l'état basculé vers le haut, par friction ou par encliquetage.

8. Appareil de mesure pour urine selon l'une des revendications 1-7, **caractérisé en ce que** le support (25) et un embout d'entrée d'écoulement (36) de la poche de collecte (33) sont pourvus de moyens de verrouillage (37, 38) par lesquels l'embout d'entrée d'écoulement (36) peut, moyennant une rotation de moins de 120° autour de son axe, être verrouillé sur le tube de liaison (23).
